# EUROPEAN PATENT SPECIFICATION

(11) **EP 2 168 955 B1**
(45) Date of publication and mention of the grant of the patent: **05.03.2014**
(21) Application number: 08776987.3
(22) Date of filing: 29.05.2008
(51) Int. Cl.: C07D 307/30, A61K 31/341, A61P 25/20

(54) **SLEEP-IMPROVING AGENT**
SCHLAFFÖRDERNDES MTTEL
AGENT D'AMÉLIORATION DU SOMMEIL

(30) Priority: 29.05.2007 JP 2007141608
(43) Date of publication of application: 31.03.2010
(60) Divisional application: 13175827.8
(73) Proprietor: Osaka Bioscience Institute, Suita-shi, Osaka 565-0874 (JP); The Doshisha, Kyoto-shi, Kyoto 602-8580 (JP); Kurei Okinawa CO., LTD., Okinawa 902-0065 (JP)
(72) Inventor: URADE, Yoshihiro, Kyoto-shi Kyoto 606-0804 (JP); HUANG, Zhi-Li, Suita-shi Osaka 565-0874 (JP); WADA, Masashi, Suita-shi Osaka 565-0874 (JP); UCHIYAMA, Nahoko, Kyotanabe-shi Kyoto 610-0395 (JP); KONISHI, Tenji, Kyotanabe-shi Kyoto 610-0395 (JP); NAKAMURA, Norio, Kyotanabe-shi Kyoto 610-0395 (JP)
(74) Representative: Zacco Denmark A/S
(86) International application number: PCT/JP2008/059925
(87) International publication number: WO 2008/146883

(56) References cited:
- WO-A1-01/58435
- WO-A1-97/42183
- WO-A1-2007/013581
- JP-A- 11 158 077
- JP-A- 2005 206 597
- M.D. GROVE ET AL.: "Pinnatine and Oxypinnatine. Novel Amino Acid Amides from Staphylea Pinnata L." TETRAHEDRON, vol. 29, 1973, pages 2715-2719, XP002586392
- GANENKO T., SEMENOV A.A., ZINCHENKO S.V.: 'Oxypinnatanine from roots of Stellaria dichotoma L' RASTITEL'NYE RESURSY vol. 38, no. 3, 2002, pages 67 - 70
- GREBNEVA N.Y., LESIOVSKAYA E.E, KHARITONOVA N.P.: 'Some pharmacologcial properties of aqueous extracts of roots of Stellaria dichotoma' RASTITEL'NYE RESURSY vol. 32, no. 4, 1996, pages 53 - 59
- INOUE TOMOHIRO ET AL.: 'Novel 2,5-dihydrofuryl-gamma-lactam derivatives from Hemerocallis fulva L. var. kwanzo Regel' CHEMICAL & PHARMACEUTICAL BULLETIN vol. 38, no. 11, 1990, pages 3187 - 3189, XP008127343
- KRUGER G.J. ET AL.: 'The Structure of N5-(3-Hydroxymethyl-2,5-Dihydro-2-Furyl)-L- Allo-gamma-Hydroxyglutamine, A New Amino Acif from HEMEROCALLIS FULVA L. (DAY LILY)' JOURNAL OF THE SOUTH AFRICAN CHEMICAL INSTITUTE vol. XXIX, 1976, pages 24 - 29, XP008127345

## Description

The present invention relates to a novel sleep-improving agent, and more particularly to a sleep-improving agent that improves sleep.

In modern society, the number of people who suffer from insomnia has increased due to stress and 24-hour lifestyles. According to a report from "Kenko Zukuri no Tameno Suimin Shishin Kentokai (Conference on Guidelines for Sleep for Better Health)", which was launched in 2003 by the Ministry of Health, Labor and Welfare, the percentage of "people who suffer from insomnia" has reached 21.4%, posing a serious problem. Accordingly, the number of people seeking a comfortable sleep will likely further increase. Along with it, the demand for a drug to treat insomnia will also likely increase in the future. Various sleeping pills are used today. However, many of these pills induce side effects such as headaches, uncomfortable feelings after awakening, physical dependency, etc. Thus, these pills do not always provide natural sleep. Accordingly, the development of a drug that can provide a more comfortable sleep is of importance.

Among crude drugs and Chinese herbal drugs that have long been used in Japan and China are many that are prescribed to treat insomnia. It is known in Japan, particularly in Okinawa, that the roots and leaves of daylilies in the Liliaceae family (*Hemerocallis fulva L. var. sempervirens*) are consumed as folk remedies for insomnia. The daylily is commonly called *nibuigusa* (*nibuigusa* means a grass that induces sleep), and the raw roots and leaves thereof are taken for insomnia, or after infusing them. A sleep-increasing effect of this plant when dried powder thereof is orally ingested by mice has been reported (Non-Patent Document 1). Further, Patent Document 1 states that a fermented material resulting from fermentation of daylilies has a sleep-improving effect. However, these reports do not determine what active ingredient contained in the plant regulates sleep.
[Non-Patent Document 1] Uezu, E. "Effect of Hemerocallis on sleep in mice" Psychiatry Clin. Neurosci. 1998, 52(2), 136-137.
[Patent Document 1] Japanese Unexamined Patent Publication No. 2006-62998

WO-A-97/42183 relates to benzofuryl derivatives and their pharmaceutically acceptable salts in the control or prevention of ilinesses or disorders of the central nervous system such as migraine, schizophrenia, anxiety states, sleep disorders, anorexia, Alzheimer's disease, addictions (alcohol, nicotine, benzodiazepine, cocaine, etc.), as well as disorders which result from damage to the head/brain or to the spinal column/bone marrow and, respectively, for the production of corresponding medicaments.

Grove M. D.; Weisleder D.; Daxenbichler M. E, Tetrahedron, Elsevier Science Publishers, Amsterdam, Vol. 29, Pages 2715 - 2719, relates to Pinnatine and Oxypinnatine obtained from Staphylea Pinnata L.

An object of the present invention is to provide a novel sleep-improving agent and a sedative agent.

The inventors of the present invention studied active ingredients in order to achieve the above object, and found that oxypinnatanine, i.e., a component of Liliaceae family (*Hemerocallis fulva L. var. sempervirens*) daylilies that grow naturally in Okinawa, has a sedative effect as well as an effect of improving sleep efficiency. The present invention is accomplished based on such a finding.

Specifically, the present invention relates to the sleep-improving agent and sedative agent described below.
Item 1. Oxypinnatanine or its acid addition salt represented by the following chemical formula I for use for improving sleep efficiency: 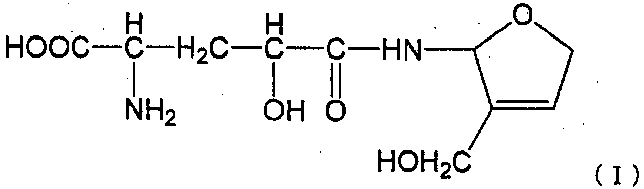
Item 2. Oxypinnatanine or its acid addition salt represented by the following chemical formula I for use for sedation:

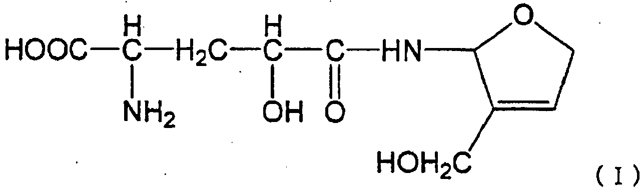

The present invention is capable of improving sleep through the ingestion of oxypinnatanine, which is a component of daylilies. In particular, the sleep-improving agent of the present invention is capable of significantly increasing non-REM sleep. Further, the sleep-improving agent of the present invention has an excellent sedative effect in addition to an excellent sleep-improving effect, and is thus also effective as a sedative agent.
[Fig. 1] A view showing the effect of oxypinnatanine for non-REM sleep (NREM sleep) when 100 mg/kg of oxypinnatanine was administered to rats, as compared to when only the vehicle was administered.
[Fig. 2] A view showing a change in the amount of locomotion (A) and the total amount of locomotion (B) after 100 mg/kg of oxypinnatanine was administered to mice, as compared to when only the vehicle was administered.
[Fig. 3] A view showing a change in the amount of sleep and wakefulness when 100 mg/kg of oxypinnatanine was administered to mice, as compared to when only the vehicle was administered.

In the present invention, the "sleep-improving agent" refers to an agent used for achieving a good sleeping condition by inducing good sleep by the effects of improving drowsiness upon awakening, facilitating sleep-onset, decreasing the number of awakenings during sleep, and the like. Further, in the present invention, the "sedative agent" refers to an agent used for relaxing humans and animals, or for improving their sleep by sedating them.

A sleep-improving agent for use in the present invention contains, as an active ingredient, oxypinnatanine or its acid addition salt represented by the following chemical formula (I):

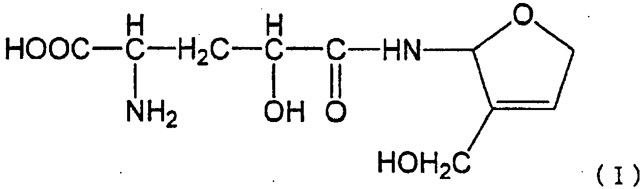

There is no limitation on the method of producing oxypinnatanine or its acid addition salt of the present invention; however, the oxypinnatanine or its acid addition salt can, for example, be prepared by extraction and isolation of plants, specifically daylilies (*Hemerocallis fulva L. var. sempervirens*), which are medicinal plants in Okinawa, used as raw materials.

When oxypinnatanine is isolated and prepared from daylilies, the daylilies as raw materials may be used wholly or partially. There is no limitation on the preparation of the oxypinnatanine; however, it can, for example, be prepared through processes such as extracting the whole plant or a portion of the daylilies using solvents including organic solvents, and fractionating the same.

For example, the whole raw daylily is cut into pieces, and extracted using an organic solvent at room temperature for 5 days to 3 weeks. Then, the extract is filtered, and the filtrate is condensed. The resulting solvent extract is separated by combining various column chromatographies, thereby obtaining the oxypinnatanine of the present invention. Examples of organic solvents include methanol, ethanol, butanol, acetone, ethyl acetate, chloroform, etc. Preferable organic solvents include methanol, ethanol, butanol, chloroform, etc. Further, extraction may be carried out using water or a mixture of water and the aforementioned organic solvents. In addition, examples of column chromatography include open column chromatography, high-performance liquid chromatography (HPLC), recycle-preparative HPLC, etc. These types of column chromatography may be combined with various carriers for column chromatography (normal phase silica gel, reversed-phase silica gel (ODS), gel filtration (Sephadex), ion-exchange resin (Diaion HP-20), etc.). Preferable combinations of column chromatography are as follows: open column chromatography combined with ion-exchange resin (Diaion HP-20), normal phase silica gel, reversed-phase silica gel (ODS), gel filtration (Sephadex, GS), etc.; recycle-preparative HPLC combined with gel filtration (Sephadex, GS); HPLC combined with reversed phase silica gel (ODS); etc.

The sleep-improving agent for use in the present invention may contain only oxypinnatanine or its acid addition salt represented by the above chemical formula (I). Additionally, other components such as carriers, substrates, or additives may be contained according to the type of usage. The composition ratio of oxypinnatanine or its acid addition salt represented by the above chemical formula (I) in the sleep-improving agent of the present invention varies depending on the type of usage of the sleep-improving agent, the level of expected effect, the sex and age of the user, etc. However, as one example, the composition ratio of oxypinnatanine or its acid addition salt represented by the above chemical formula (I) is 0.01 to 100 wt.%, preferably 0.1 to 10 wt.%, of the total weight of the sleep-improving agent.

The sleep-improving agent for use in the present invention may be used as a pharmaceutical product, food, etc.

The administration and dosage form of the sleep-improving agent for use in the present invention may be either an oral administration form or a parenteral administration form. Examples of oral administration forms include solid forms such as powders, granules, capsules, tablets, chewable tablets, etc., and liquid forms such as solutions, syrups, etc. Examples of parenteral administration forms include injections, sprays, etc. A preferable administration form is oral administration by tablets, capsules, etc..

The sleep-improving agent for use in the present invention can be formulated into a drug using a known formulation method, specifically, a formulation technology suitable for oral ingestion.

For example, when the sleep-improving agent for use in the present invention is used as a pharmaceutical product, such a pharmaceutical composition can be produced by uniformly mixing, as an active ingredient, an effective amount of oxypinnatanine or its acid addition salt in the form of isolation or an acid addition salt with a pharmaceutically acceptable carrier. Such a carrier can be produced in a wide range of forms according to the form of pharmaceutical preparation suitable for administration. Preferably, these pharmaceutical compositions are present in a unit dosage form suitable for oral administration. In the preparation of a composition in the oral dosage form, any carrier that is effective and pharmacologically acceptable may be used. For example, oral liquid preparations such as suspensions and syrups may be produced by using water; sugars such as sucrose, sorbitol and fructose; glycols such as polyethylene glycol and propylene glycol; oils such as sesame oil, olive oil, and soybean oil; preservatives such as alkyl para-hydroxybenzoate; and flavors such as strawberry flavor and peppermint.

Powders, pills, capsules, and tablets may be produced by using excipients such as lactose, glucose, sucrose, and mannitol; disintegrants such as starch and sodium alginate; lubricants such as magnesium stearate and talc; binders such as polyvinyl alcohol, hydroxypropyl cellulose, and gelatin; surfactants such as fatty acid ester; and plasticizers such as glycerin. Tablets and capsules are the most useful oral unit dosage forms because they are easy to administrate. A solid pharmaceutical carrier is used in the production of tablets and capsules.

In the case of oral administration of the sleep-improving agent for use in the present invention, the effective dosage varies depending on the age, weight, and clinical condition of the patient, administration method, and the like. However, the agent is usually administered such that an active ingredient (oxypinnatanine or its derivative) amounts to about 1 to 10,000 mg/kg/day, preferably about 10 to 1,000 mg/kg/day. Additionally, the agent should normally be administered 1 to 6 hours before sleep, more preferably 2 to 5 hours before sleep.

### [Examples]

Hereinafter, the present invention will be described in detail by referring to the following descriptions: an example of production of oxypinnatanine, which is an active ingredient of the sleep-improving agent of the present invention; examples for clarifying the effects of the sleep-improving agent and the sedative agent of the present invention; and preparation examples of the sleep-improving agent of the present invention. However, the present invention is not limited to these Examples and the like.

### Example 1

### Method of Producing Oxypinnatanine

The whole raw daylily was cut into pieces, and extracted using methanol as a solvent at room temperature for one week. Then, the extract was filtered, and the filtrate was condensed. The resulting solid (hereinafter referred to as "methanol extract") was isolated using ion-exchange resin (Diaion HP-20), normal phase silica gel, and gel filtration (Sephadex, GS) for open column chromatography, and using gel filtration (Sephadex, GS) for recycle-preparative HPLC to obtain oxypinnatanine.

The results of data obtained for oxypinnatanine are as follows:
·Colorless needle crystal (H₂O-MeOH); mp 152-153°C (decomp.); IR(KBr)vₘₐₓ 3350, 1660, 1615, 1504 cm⁻¹.
·¹H NMR (D₂O, 400MHz) δ: 6.40 (1H, m, 2'), 6.19 (1H, ddd, J=4, 4, 2Hz, 4'), 5.63 (1H, dddd, J=14, 4, 4, 2Hz, 5'), 4.74 (1H, dddd, J=14, 4, 4, 2 Hz, 5'), 4.34 (1H, dd, J=9, 4 Hz, 3), 4.26 (1H, dd, J=14, 2 Hz, 6'), 4.18 (1H, dd, J=14, 2 Hz, 6'), 3.98 (1H, dd, 7, 4 Hz, 5), 2.34 (1H, ddd, J=15, 7, 4 Hz, 4), 2.22 (1H, ddd, J=15, 9, 4 Hz, 4).
·¹³C NMR (D₂O, 100 MHz) δc: 178.7(2), 175.9(6), 138.6(3'), 129.1(4'), 87.5(2'), 76.6(5'), 71.7(3), 58.9(6'), 55.2(5).

### Test Example 1

### 1. Method

### 1) Used Animals

Sprague-Dawley rats (male, 8 weeks old, weighing 250-280 g) were purchased from Japan SLC, Inc.

### 2) Feeding Method

The rats were individually housed in acrylic cages placed in a sound insulation chamber. They were given solid rat food (food name: Labo MR Stock) in a 12-hour light and dark cycle (light period starting at 7 a.m.), with free access to food and drinking water.

### 3) Operation to Implant Electrodes for Measuring EEG/EMG and Connection to Measuring Device

An operation to implant electrodes for measuring EEG/EMG was performed on the rats (Huang Z. L. et al., J. Neurosci. 2003, 23(14), 5975-83., Okada T. et al., Biochem. Biophys. Res. Commun. 2003, 312(1), 29-34). The rats were placed in a chamber for recovery for 10 days, and recovered. Then, the rats were moved to a chamber for recording, and measurement cables were connected to the electrodes. The rats were adapted to the environment for 4 days.

### 4) Sample Administration

Oxypinnatanine was dissolved in water, and a dosage of 100 mg/kg was orally administered using a probe. Administration was carried out at 19:00 (starting time of a dark period). On day 1, water was solely administered as a control containing only the solvent. On day 2, oxypinnatanine was administered (n=6).

### 5) Recording and Analysis of EEG/EMG

The EEG and EMG were amplified (EEG: 0.5-30 Hz, EMG: 20-200 Hz), and then digitalized at a sampling speed of 128 Hz for recording. For analysis, EEG recording software "Sleep Sign" (Kissei Comtec) was used to automatically determine each epoch (10 seconds) of data as wakefulness, non-REM sleep, or REM sleep based on the frequency components and waveforms of EEG and EMG. The obtained determination results were ultimately checked by the experimenters themselves, and corrected as needed. EEG data over 12 hours after administration were analyzed. Then, waking time, non-REM sleep time, and REM sleep time per hour were calculated. Further, the EEG power spectrum was analyzed, and the amplitudes of theta waves and delta waves were thereby analyzed.

### 2. Result

### Total Non-REM Sleep Time during 6 Hours after Administration

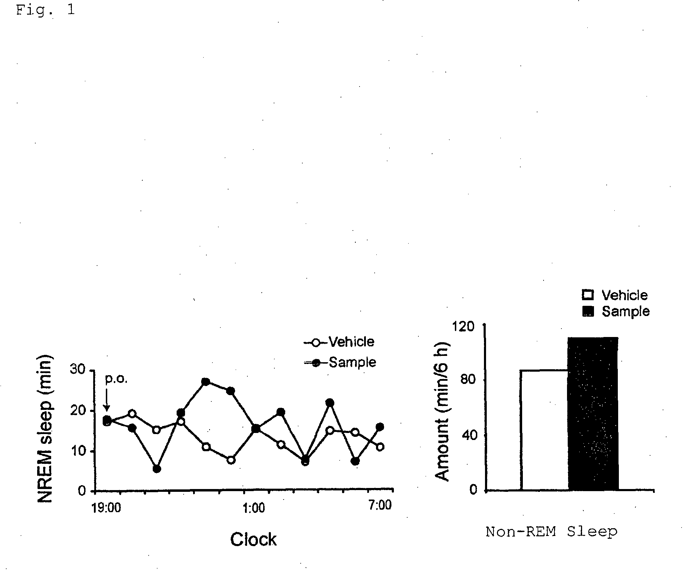

The dosage of 100 mg/kg exhibited an effect of increasing non-REM sleep time compared to when the vehicle (water) was administered (Fig. 1).

### Test Example 2

### 1. Method

### 1) Used Animals

Slc:ICR mice (male, 10 weeks old, weighing 38-40 g) were purchased from Japan SLC, Inc.

### 2) Feeding Method

The mice were housed in groups (8 mice per group) in acrylic cages placed in a chamber for monitoring the amount of animal behavior, and were habituated for 7 days. They were given solid rat food (food name: Labo MR Stock) in a 12-hour light and dark cycle (light period starting at 7 a.m.), with free access to food and drinking water.

### 3) Sample Administration

Oxypinnatanine was dissolved in water, and a dosage weight of 100 mg/kg was orally administered using a probe. Administration was carried out at 19:00 (starting time of a dark period). On day 1, water was solely administered as a control containing only the solvent. On day 2, oxypinnatanine was administered (n=8).

### 4) Recording and Analysis of Locomotor Assay (Amount of Locomotion)

An infrared monitor was used to detect infrared light emitted from the animals to count the amount of locomotion. The infrared monitor recorded continuously for 12 hours. Recording was conducted using a Biotex 16CH Act Monitor BAI2216 (Biotex). For analysis, the time-dependent change in the amount of locomotion and the total amount of locomotion in each hour were calculated.

### 2. Result

### Total Amount of Locomotion during 6 Hours after Administration

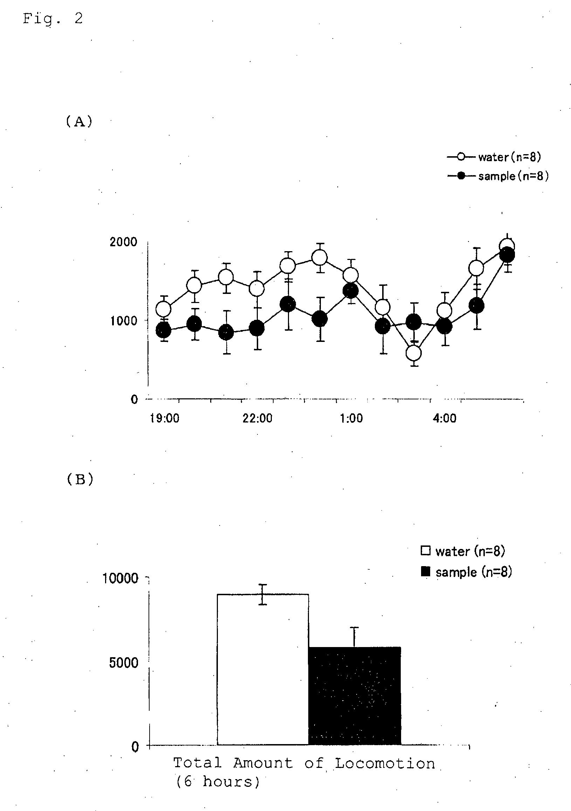

The dosage of 100 mg/kg exhibited a statistically significant decrease in the amount of locomotion compared to when the vehicle (water) was administered (Fig. 2).

### Test Example 3

### 1. Method

### 1) Used Animals

C57BL/6 mice (male, 10 weeks old, weighing 25-28 g) were purchased from Oriental BioService Inc.

### 2) Feeding Method

The mice were individually housed in acrylic cages placed in a sound insulation chamber. They were given solid mouse food (food name: Labo MR Stock) in a 12-hour light and dark cycle (light period starting at 8 a.m.), with free access to food and drinking water.

### 3) Operation to Implant Electrodes for Measuring EEG/EMG and Connection to Measuring Device

An operation to implant electrodes for measuring EEG/EMG was performed on the mice. The mice were placed in a chamber for recovery for 10 days, and recovered. Then, the mice were moved to a chamber for recording, and measurement cables were connected to the electrodes. The mice were adapted to the environment for 4 days.

### 4) Sample Administration

Oxypinnatanine was dissolved in water, and a dosage of 10 mg/kg was orally administered using a probe. Administration was carried out at 20:00 (starting time of a dark period). On day 1, water was solely administered as a control containing only the solvent. On day 2, oxypinnatanine was administered (n=4).

### 5) Recording and Analysis of EEG/EMG

The EEG and EMG were amplified (EEG: 0.5-30 Hz, EMG: 20-200 Hz), and then digitalized at the sampling speed of 128 Hz for recording. For analysis, EEG recording software "Sleep Sign" (Kisse Comtec) was used to automatically determine each epoch (10 seconds) of data as wakefulness, non-REM sleep, or REM sleep based on the frequency components and waveforms of EEG and EMG. The obtained determination results were ultimately checked by the experimenters themselves, and corrected as needed. EEG data over 12 hours after administration were analyzed. Then, waking time, non-REM sleep time, and REM sleep time per hour were calculated. Further, the EEG power spectrum was analyzed, and the amplitudes of theta waves and delta waves were thereby analyzed.

### 2. Result

### Total Non-REM Sleep Time during 4 Hours after Administration

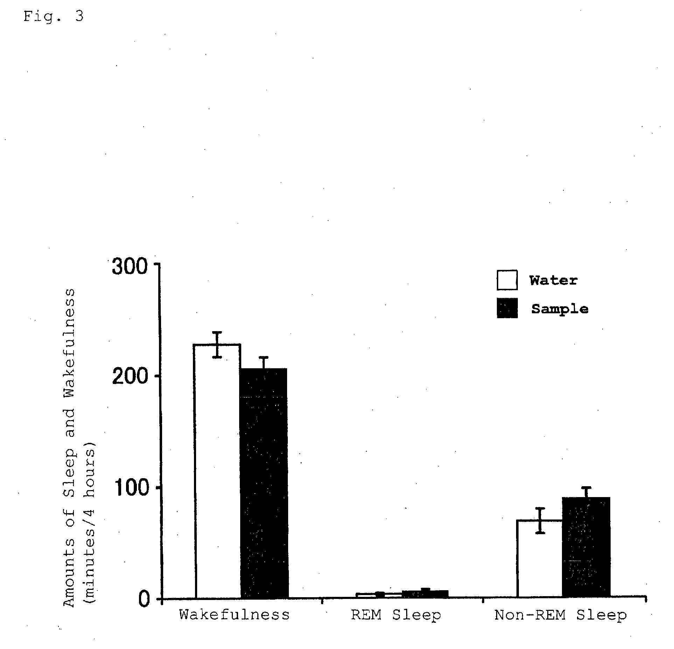

The dosage of 10 mg/kg exhibited an effect of increasing non-REM sleep time with statistical significance, compared to when the vehicle (water) was administered (Fig. 3).

### Preparation Example 1: Tablets

Tablets are prepared with the following composition by conventional procedures.

| | |
|---|---|
| Oxypinnatanine | 200 mg |
| Lactose | 60 mg |
| Potato starch | 30 mg |
| Polyvinyl alcohol | 2 mg |
| Magnesium stearate | 1 mg |
| Tar dye | trace |

### Preparation Example 2: Powders

Powders are prepared with the following composition by conventional procedures.

| | |
|---|---|
| Oxypinnatanine | 200 mg |
| Lactose | 275 mg |

### [Industrial Applicability]

The oxypinnatanine of the present invention can be used as a sleep-improving agent and a sedative agent.

## Claims

1. Oxypinnatanine or its acid addition salt represented by the following chemical formula I for use for improving sleep efficiency:

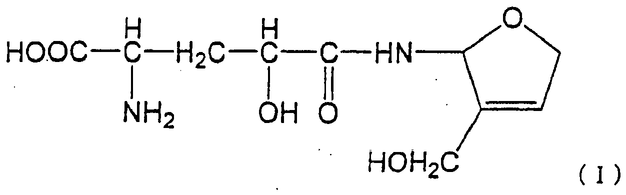

2. Oxypinnatanine or its acid addition salt represented by the following chemical formula I for use for sedation:

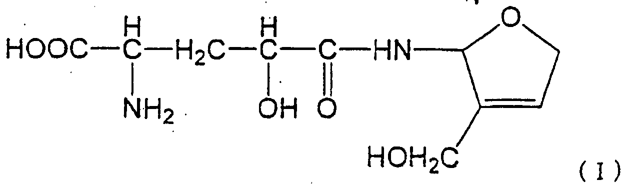

## Patentansprüche

1. Oxypinnatanin oder dessen Säureadditionssalz, vertreten durch die folgende chemische Formel I, zur Anwendung zur Verbesserung der Schlafeffizienz:

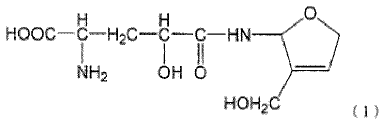

2. Oxypinnatanin oder dessen Säureadditionssalz, vertreten durch die folgende chemische Formel I, zur Anwendung für Sedierung:

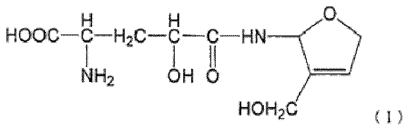

## Revendications

1. Oxypinnatanine ou son sel d'addition d'acide représenté par la formule chimique I suivante pour l'utilisation dans l'amélioration de l'efficacité du sommeil:

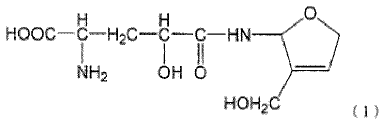

2. Oxypinnatanine ou son sel d'addition d'acide représenté par la formule chimique suivant pour l'utilisation dans la sédation:
